# EUROPEAN PATENT APPLICATION

(11) **EP 4 005 464 A1**
(43) Date of publication of application: **01.06.2022**
(21) Application number: 20846667.2
(22) Date of filing: 31.07.2020
(51) Int. Cl.: A61B 3/13

(54) **CLOSE-UP PHOTOGRAPHIC DEVICE**

(30) Priority: 31.07.2019 JP 2019140855
(71) Applicant: Oui Inc., Tokyo 160-0022 (JP)
(72) Inventor: SHIMIZU, Eisuke, Tokyo 160-0022 (JP); YAZU, Hiroyuki, Tokyo 160-0022 (JP); AKETA, Naohiko, Tokyo 160-0022 (JP); YOKOIWA, Ryota, Tokyo 160-0022 (JP); NAKAYAMA, Shintaro, Tokyo 160-0022 (JP); SAKASEGAWA, Akito, Tokyo 160-0022 (JP)
(74) Representative: Mewburn Ellis LLP
(86) International application number: PCT/JP2020/029578
(87) International publication number: WO 2021/020584

(57) **Abstract**

To provide a close-up imaging device whereby observation and imaging of an internal state or a surface state of anterior eye can easily be performed by attachment to a mobile terminal such as a smartphone.

The above-described problem is solved by a close-up imaging device (10) detachably mounted to a mobile terminal (50) equipped with a light source (52) and an imaging camera lens (53). The close-up imaging device (10) is an integral structure comprising at least a convex lens (2), a slit light forming part (3), and a color filter (4). When mounted to the mobile terminal (50), the close-up imaging device (10) is configured so that the convex lens (2) is positioned in front of the imaging camera lens (52), the slit light forming part (3) is positioned in front of the light source (52), and the color filter (4) is installed or removed from in front of the light source (52).

## Description

### Field of the Invention

The present invention relates to a close-up imaging device utilized for diagnosing a lesion by observing or capturing an image of an internal state or a surface state of an anterior eye or the like, and used by being mounted to a portable mobile terminal, such as a smartphone.

### BACKGROUND ART

To perform an eye examination, a specialized magnifier is required. A doctor carries out a process of performing an examination using the magnifier, obtaining findings to conduct an evaluation, and making a diagnosis to provide treatment. In an ophthalmic outpatient clinic, a slit lamp microscope is used. However, because the slit lamp microscope is large, heavy, and expensive, a handheld slit lamp microscope has been widely used when simplicity is required or at an ophthalmic examination site other than an outpatient clinic, such as bedside.

However, with a handheld slit lamp microscope, operation is difficult and therefore the information obtained is less and, while it is possible to observe the anterior eye, it is impossible to record images. Further, with a handheld slit lamp microscope, it takes time to obtain findings. Furthermore, the number of patients or the like that can be examined at one time is only one. In addition, the handheld slit lamp microscope has many problems such as being heavy due to inclusion of a light source.

As prior art for solving the above-described problems, for example, close-up imaging devices and ultra-close-up imaging devices, each having a lighting function and capturing an image with a smartphone fixed onto a stand with a light source have been proposed (refer to, for example, Patent Documents 1 and 2). Further, for example, ophthalmoscopes including a system or an application for storing an image obtained on the basis of an image acquiring technique have been proposed (refer to, for example, Patent Document 3). Furthermore, for example, wide-field retinal image acquiring systems and methods for capturing and analyzing an image of a retina using a smartphone have been proposed (refer to, for example, Patent Document 4).

### PRIOR ART DOCUMENTS

### Patent Documents

Patent Document 1: Japanese Laid-Open Patent Application Publication No. 2017-121320
Patent Document 2: Japanese Unexamined Utility Model Registration Application Publication No. 3197418
Patent Document 3: Japanese Unexamined Patent Application Publication No. 2016-524483
Patent Document 4: Japanese Unexamined Patent Application Publication No. 2017-501005

### SUMMARY OF THE INVENTION

### Problems to be Solved by the Invention

However, the devices described in the above-described Patent Documents 1 and 2 remain problematic in that they are heavy due to inclusion of a light source, do not permit easy observation of both the interior and the surface of the anterior eye, and the like. Further, the device described in the above-described Patent Document 3 is problematic in that it does not have a close-up function during imaging of the anterior eye, details of the light source are not described and thus an image cannot actually be acquired, and the like. Furthermore, the device described in the above-described Patent Document 4 is problematic in that it puts only an image of the retina into focus, imaging of the anterior eye is not performed, and the like.

Further, the conventional devices described in each of the patent documents described above cannot obtain findings unless the examination is performed by a skilled person. Furthermore, macro problems such as evaluations having to be performed at the examination site, and problems such as devices not being able to be used for research remain. In addition, none of the devices described in each of the patent documents described above has been commercialized.

The present invention has been made to solve the above-described problems, and an object thereof is to provide a close-up imaging device utilized for diagnosing a lesion by observing or capturing an image of an internal state or a surface state of an anterior eye or the like, and used by being mounted to a portable mobile terminal, such as a smartphone.

### Means for Solving the Problems

A close-up imaging device according to the present invention is a close-up imaging device detachably mounted to and used with a mobile terminal equipped with a light source and an imaging camera lens and, when the close-up imaging device is mounted to the mobile terminal, is characterized in that a convex lens is positioned in front of the imaging camera lens, and a slit light forming part or a color filter is positioned in front of the light source. The close-up imaging device according to first and second aspects having such characteristics is as follows.

### (First aspect)

A close-up imaging device according to the present invention is a close-up imaging device detachably mounted to and used with a mobile terminal equipped with a light source and an imaging camera lens, and is an integral structure comprising at least a convex lens, a slit light forming part, and a color filter, and, when the close-up imaging device is mounted to the mobile terminal, the convex lens is a member positioned in front of the imaging camera lens, the slit light forming part is a member positioned in front of the light source, and the color filter is a member installed or removed from in front of the light source.

According to this invention, it is possible to form slit light by the slit light forming part and, with the formed slit light reaching the eye, perform observation and imaging of an internal state of an anterior eye in more detail. By observing the anterior eye through the color filter, it is possible to easily observe an injury of the anterior eye and the like. Further, it is possible to perform such observation and imaging with a mobile terminal such as a smartphone, and thus observe or capture an image of the internal state and the surface state of the anterior eye not just in a hospital, and utilize the device for diagnosis of lesions and the like.

In the close-up imaging device according to the present invention, the color filter is a member removed from in front of the light source during imaging or observation of an anterior eye, and installed in front of the light source during observation or imaging of an injury of an anterior eye. According to this invention, the color filter can be installed or removed from in front of the light source as needed.

In the close-up imaging device according to the present invention, the integral structure includes a base plate positioned on the mobile terminal side, and the color filter also serves as the base plate or is provided as a portion of the base plate, and when slit light is to be formed by the slit light forming part, the color filter is slid to be removed from an optical path of the light source, and the slit light forming part is slid to be installed in the optical path, and when slit light is not to be formed by the slit light forming part, the color filter is slid to be installed in the optical path, and the slit light forming part is slid to be removed from the optical path. According to this invention, the color filter and the slit light forming part can be installed in or removed from the optical path by respectively sliding the color filter and the slit light forming part.

In the close-up imaging device according to the present invention, the slit light forming part is a member that forms light from the light source into slit light by a cylindrical lens. According to this invention, the slit light can be readily formed and emitted onto the eye to observe or capture an image of the internal state of the anterior eye.

In the close-up imaging device according to the present invention, the slit light forming part includes a first reflecting mirror that reflects the light from the light source, a second reflecting mirror that reflects light reflected by the first reflecting mirror, and a slit part that allows light reflected by the second reflecting mirror to pass therethrough, and is a member that forms light that passes through the slit part into slit light by the cylindrical lens. According to this invention, the light reflected by the two reflecting mirrors passes through the slit part, making it possible to form the slit light.

The close-up imaging device according to the present invention further comprises a slide member for sliding the cylindrical lens in a direction parallel to the base plate and in a direction orthogonal to the cylindrical lens. According to this invention, by sliding the cylindrical lens, it is possible to adjust an incident form in which the slit light is incident on the eye.

### (Second aspect)

A close-up imaging device according to the present invention is a close-up imaging device detachably mounted to and used with a mobile terminal equipped with a light source and an imaging camera lens, and comprises a first structure including a convex lens and a slit light forming part, and a second structure including a convex lens and a color filter, as interchangeable members, and an engaging block detachably mounted to the mobile terminal and for engaging the interchangeable members, and, when the close-up imaging device is mounted to the mobile terminal, the convex lens is positioned in front of the imaging camera lens, the slit light forming part is positioned in front of the light source, and the color filter is installed or removed from in front of the light source.

According to this invention, the first structure including the slit light forming part and the second structure including the color filter are provided as interchangeable members and each is mounted by being engaged with the engaging block, making it possible to form slit light by the slit light forming part of the first structure and, with the formed slit light reaching the eye, perform observation and imaging of an internal state of an anterior eye in more detail. Further, by observing the anterior eye through the color filter of the second structure, it is possible to easily observe an injury of the anterior eye and the like. Furthermore, it is possible to perform such observation and imaging with a mobile terminal such as a smartphone, and thus observe or capture an image of the internal state and the surface state of the anterior eye not just in a hospital, and utilize the device for diagnosis of lesions and the like.

In the close-up imaging device according to the present invention, the slit light forming part is a member that forms light from the light source into slit light by a cylindrical lens.

In the close-up imaging device according to the present invention, the slit light forming part includes a first reflecting mirror that reflects the light from the light source, a second reflecting mirror that reflects light reflected by the first reflecting mirror, and a slit part that allows light reflected by the second reflecting mirror to pass therethrough, and is a member that forms light that passes through the slit part into slit light by the cylindrical lens.

In the close-up imaging device according to the present invention, the slit light forming part further includes a slide function capable of sliding in accordance with a distance between the light source of the mobile terminal and the imaging camera lens.

In the close-up imaging device according to the present invention, the second structure further includes an opening window on the light source, and the color filter is a member removed from the opening window during imaging or observation of an anterior eye, and installed in the opening window during observation or imaging of an injury of an anterior eye. At this time, the color filter may be installed or removed as a slide plate or as an opening/closing door.

In the close-up imaging device according to the present invention, the engaging block includes an engaging recessed part, and the engaging recessed part engages with an engaging protruding part provided to both the first structure and the second structure.

In the close-up imaging device according to the present invention, the engaging block is a clamp engaging block mounted to the mobile terminal by a C-clamp or an adhering engaging block mounted to the mobile terminal by an adhesive member.

### Effect of the Invention

According to the present invention, it is possible to provide a close-up imaging device whereby observation and imaging of an internal state or a surface state of anterior eye can easily be performed by attachment to a mobile terminal such as a smartphone.

### BRIEF DESCRIPTION OF THE DRAWINGS

Fig. 1 is a perspective view illustrating an example of a close-up imaging device according to a first aspect of the present invention.
Fig. 2 is a perspective view illustrating a state in which the close-up imaging device is mounted to a smartphone.
Fig. 3 is an explanatory view of an internal structure of the close-up imaging device.
Fig. 4 is an explanatory view illustrating an optical path when slit light is irradiated to observe or capture an image of an internal state of an anterior eye.
Fig. 5 is an explanatory view illustrating a form in which a cylindrical lens is slid.
Fig. 6 is an explanatory view illustrating a form when white light is irradiated while being transmitted through a color filter and an injury or the like of an anterior eye is observed and captured in an image.
Fig. 7 is an explanatory view of a base plate from which a slit light forming part is removed in order to explain in detail an installation form of the base plate, Fig. 7A illustrating a form in which an opening of the base plate that also serves as the color filter is installed so as to be positioned in the optical path of a light source, and Fig. 7B illustrating a form in which the base plate that also serves as the color filter is installed so as to be positioned in the optical path of the light source.
Fig. 8 shows, in the upper row, representative photographs of an eye exposed to white light and, in the lower row, representative photographs of fluorescein staining. The images in the left column are examples of an eye captured with an existing device, and the images in the right column are examples of an eye captured using the close-up imaging device.
Figs. 9A to 9F are examples of captured images for evaluating nuclear sclerosis (cataracts) by the close-up imaging device.
Fig. 10 is a perspective view illustrating an example in which the close-up imaging device (first structure) according to a second aspect of the present invention is mounted to a mobile terminal with a clamp engaging block.
Fig. 11 is a perspective view illustrating an example in which the close-up imaging device (first structure) according to the second aspect of the present invention is mounted to a mobile terminal with an adhering engaging block.
Fig. 12 is a perspective view illustrating an example in which the close-up imaging device (second structure) according to the second aspect of the present invention is mounted to a mobile terminal with the clamp engaging block, Fig. 12A illustrating a case in which the color filter is installed, and Fig. 12B illustrating a case in which the color filter is removed.
Fig. 13 is a perspective view illustrating an example in which the close-up imaging device (second structure) according to the second aspect of the present invention is mounted to a mobile terminal with the adhering engaging block, Fig. 13A illustrating a case in which the color filter is installed, and Fig. 13B illustrating a case in which the color filter is removed.
Figs. 14A and 14B are explanatory views (Part 1) of the first structure including the slit light forming part.
Figs. 15A and 15B are explanatory views (Part 2) of the first structure including the slit light forming part.
Figs. 16A to 16C are explanatory views (Part 1) of the second structure including the color filter.
Figs. 17A and 17B are explanatory views (Part 2) of the second structure including the color filter.
Figs. 18A to 18D are explanatory views of the clamp engaging block.
Figs. 19A to 19D are explanatory views of the adhering engaging block.
Figs. 20A to 20D are explanatory views of a clamp.
Fig. 21 is a representative photograph of an eye exposed to white light by the close-up imaging device illustrated in Fig. 10.

### Embodiments of the Invention

A close-up imaging device according to the present invention will now be described with reference to the drawings. The present invention is not limited to the embodiments described below, and includes modifications and applications thereof.

### [Close-up Imaging Device]

A close-up imaging device 10 according to the present invention is a close-up imaging device detachably mounted to and used with a mobile terminal 50 equipped with a light source 52 and an imaging camera lens 53. When mounted to the mobile terminal 50, the close-up imaging device 10 according to the present invention is characterized in that a convex lens 2 is positioned in front of the imaging camera lens 53, and a slit light forming part 3 or a color filter 4 is positioned in front of the light source 52. The close-up imaging device according to first and second aspects having such characteristics is as follows.

### (Close-up imaging device of first aspect)

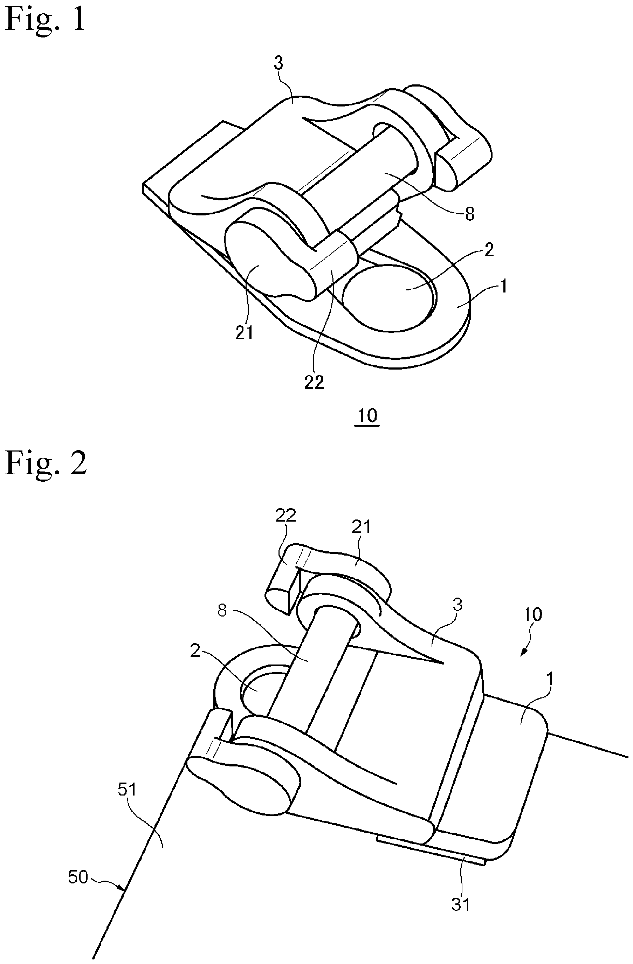

The close-up imaging device 10 according to the present invention is, as illustrated in Fig. 1 and Fig. 2, the close-up imaging device 10 detachably mounted to the mobile terminal 50 equipped with the light source 52 and the imaging camera lens 53. The close-up imaging device 10 is an integral structure including at least the convex lens 2, the slit light forming part 3, and the color filter 4. When mounted to the mobile terminal 50, the close-up imaging device 10 is configured so that the convex lens 2 is positioned in front of the imaging camera lens 53, the slit light forming part 3 is positioned in front of the light source 52, and the color filter 4 is installed or removed from in front of the light source 52.

With this close-up imaging device 10, it is possible to form slit light by the slit light forming part 3 and, with the formed slit light reaching the eye, perform observation and imaging of an internal state of an anterior eye in more detail. By observing the anterior eye through the color filter 4, it is possible to easily observe an injury of the anterior eye and the like. Further, it is possible to perform such observation and imaging with the mobile terminal 50 such as a smartphone, and thus observe or capture an image of an internal state and a surface state of the anterior eye not just in a hospital, and utilize the device for diagnosis of lesions and the like.

### (Close-up imaging device of second aspect)

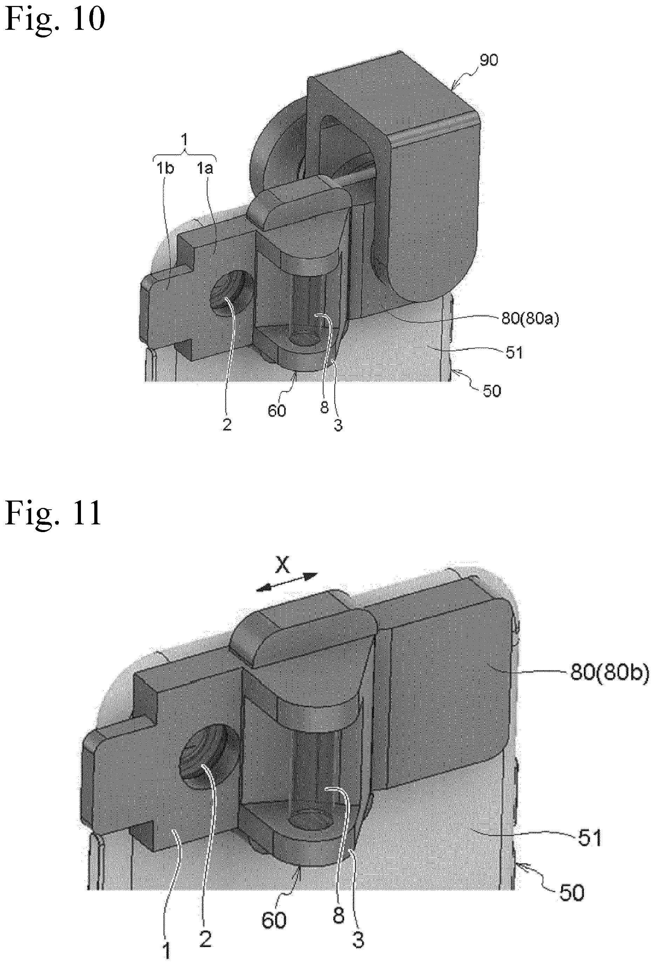

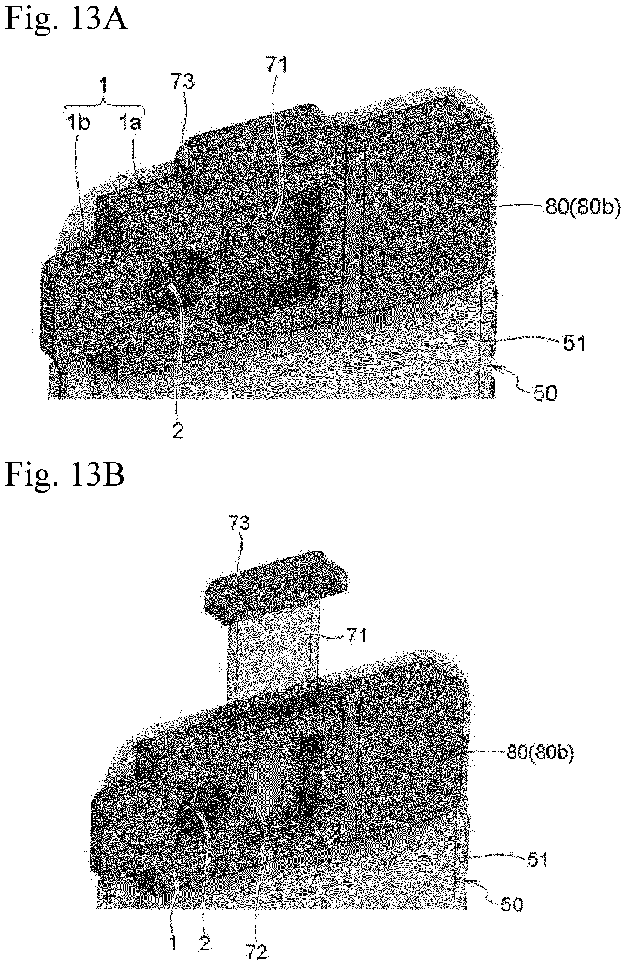

The close-up imaging device 10 according to the present invention is, as illustrated in Figs. 10 and 11, Figs. 12A and 12B, and Figs. 13A and 13B, a close-up imaging device detachably mounted to and used with the mobile terminal 50 equipped with the light source 52 and the imaging camera lens 53. The close-up imaging device 10 includes a first structure 60 including the convex lens 2 and the slit light forming part 3, and a second structure 70 including the convex lens 2 and a color filter 71, as interchangeable members, and an engaging block 80 detachably mounted to the mobile terminal 50 and for engaging the interchangeable members (first structure 60 and second structure 70). When mounted to the mobile terminal 50, the close-up imaging device 10 is configured so that the convex lens 2 is positioned in front of the imaging camera lens 53, the slit light forming part 3 is positioned in front of the light source 52, and the color filter 71 is installed or removed from in front of the light source 52.

With this close-up imaging device 10, the first structure 60 including the slit light forming part 3 and the second structure 70 including the color filter 71 are provided as interchangeable members and each is mounted by being engaged with the engaging block 80, making it possible to form slit light by the slit light forming part 3 of the first structure 60 and, with the formed slit light reaching the eye, perform observation and imaging of an internal state of an anterior eye in more detail. Further, by observing the anterior eye through the color filter 71 of the second structure 70, it is possible to easily observe injuries of the anterior eye and the like. Furthermore, it is possible to perform such observation and imaging with the mobile terminal 50 such as a smartphone, and thus observe or capture an image of the internal state and the surface state of the anterior eye not just in a hospital, and utilize the device for diagnosis of lesions and the like.

Hereinafter, each component will be described in detail. It should be noted that, in the following, the close-up imaging device of the first aspect will be referred to as the close-up imaging device of a first embodiment, and the close-up imaging device of the second aspect will be referred to as the close-up imaging device of a second embodiment. Unless otherwise specified, the components are common to both and, when described as "first embodiment" or "second embodiment," are the components of the corresponding embodiment.

### <Mobile terminal>

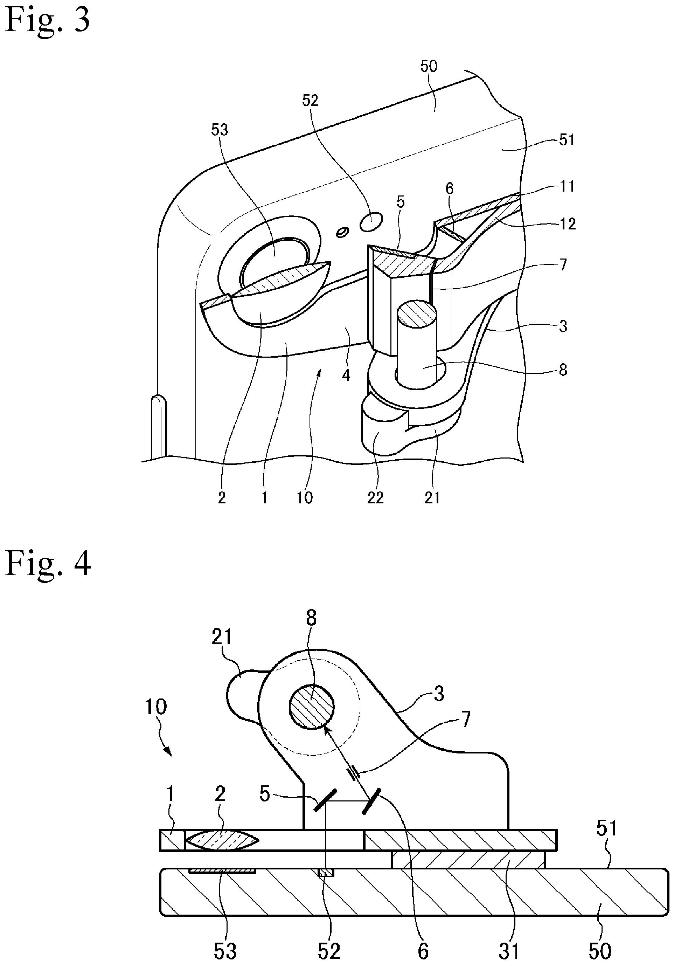

The mobile terminal 50 does not constitute the close-up imaging device 10 according to the present invention, but is an object subject to mounting to which the close-up imaging device 10 is detachably mounted. A surface 51 serving as a mounting surface of the mobile terminal 50 includes the light source 52 and the imaging camera lens 53. The close-up imaging device 10 according to the present invention is detachably mounted to the surface 51. While a smartphone is used as the mobile terminal 50 in the example in Fig. 3, the object subject to mounting to which the close-up imaging device 10 is mounted need only be the mobile terminal 50 equipped with the light source 52 and the imaging camera lens 53, and may be, for example, a tablet terminal.

The imaging camera lens 53 is often provided to a left end portion of an upper end portion of the mobile terminal (smartphone) 50, as illustrated in Fig. 3, but the position is not particularly limited. Further, the light source 52 is provided to the upper end portion of the mobile terminal (smartphone) 50, adjacent to the imaging camera lens 53. The position is not particularly limited, but it is often a portion on a right side of, a portion on a left side of, or a portion below the imaging camera lens 53.

### <Close-up imaging device of first embodiment

### (Integral structure)

As an integral structure, at least the convex lens 2, the slit light forming part 3, and the color filter 4 are integrated. "Integrated" does not mean that the objects are fixed by adhesion, welding, or the like, but rather "not separated." This is because the close-up imaging device 10 according to the present invention is a small structure that can be detachably attached to the mobile terminal 50, such as a smartphone, and thus when the convex lens 2, the slit light forming part 3, and the color filter 4 are separated from each other, the parts may be lost. It should be noted that "at least" means that a structure or a supplementary member other than the convex lens 2, the slit light forming part 3, and the color filter 4 may be included.

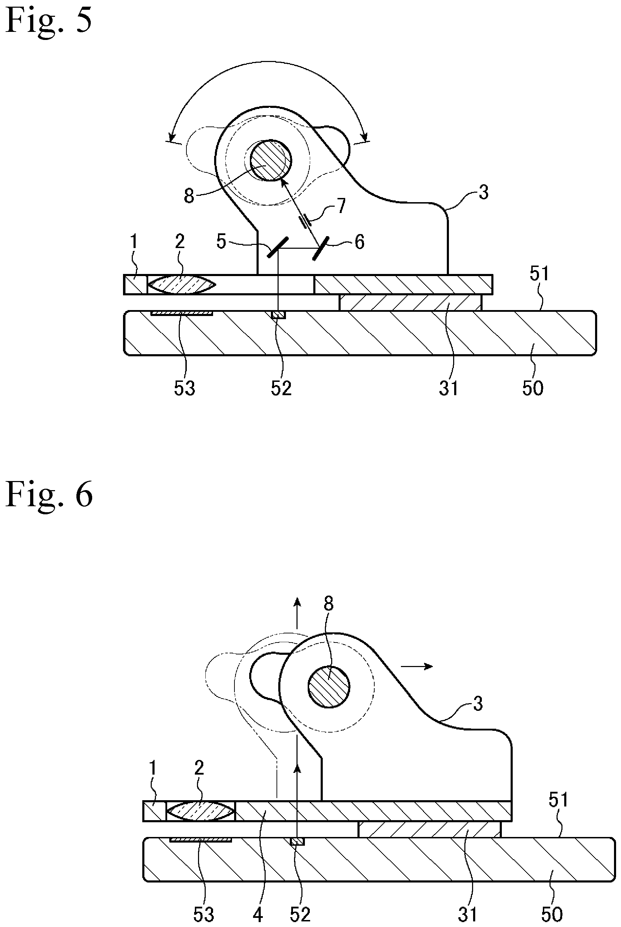

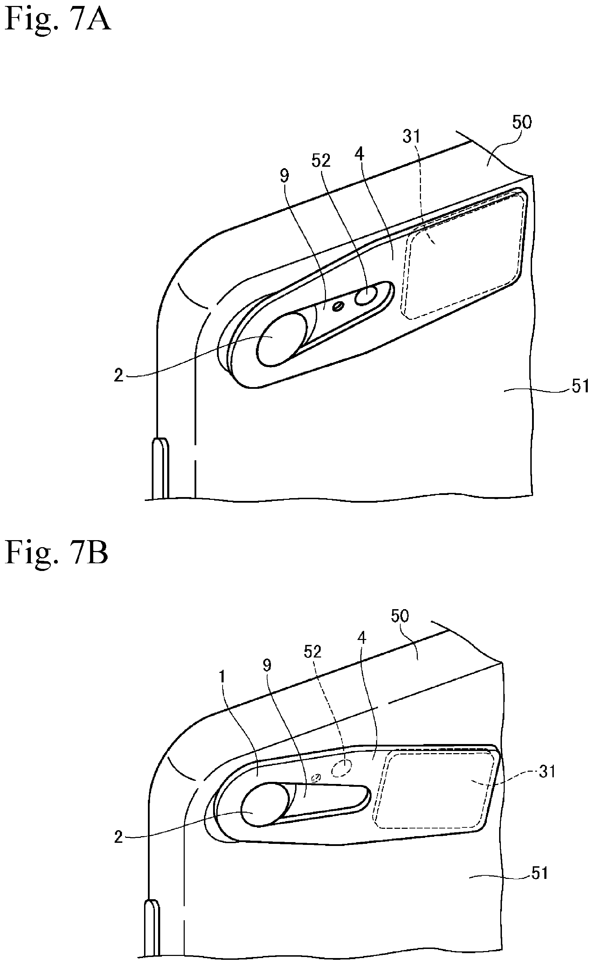

In the close-up imaging device 10 of the first embodiment, as illustrated in Fig. 1 and the like, a base plate 1 is constituted by a color filter material. This way, the base plate 1 can also serve as the color filter 4, which is preferable. Further, as illustrated in Figs. 7A and 7B, the base plate 1 is provided with an opening 9 for passing the optical path of the light source 52 from the light source 52 to pass therethrough. The convex lens 2 is adhered or fitted to and thus integrated with the opening 9. Furthermore, as illustrated in Fig. 6, the slit light forming part 3 is slidably provided to the base plate 1. A slide mechanism at this time may be (a) a mechanism in which a rail structure is provided to the base plate 1 and the slit light forming part 3 slides on the rail structure, or may be (b) a mechanism in which the slit light forming part 3 is bonded to the base plate 1 via a detachable suction sheet. As this suction sheet, a sheet that is the same as the suction sheet denoted by reference sign 31 can be used. In this way, at least the convex lens 2, the slit light forming part 3, and the color filter 4 are integrated.

The integral structure integrated is detachably attached to the surface 51 of the mobile terminal 50, and examples of attachment means thereof preferably include a mode using the suction sheet 31. It should be noted that a member other than this may be used as the detachable integral structure. It should be noted that the suction sheet 31 is also generally commercially available as a "suction sheet," and examples thereof include a sheet obtained by providing a foam layer composed of an independent foam on a base film made of polyethylene terephthalate (PET) or the like. The foam layer composed of an independent foam is adhered to the surface 51 of a smartphone, for example, by allowing air in holes to be released, like a suction cup composed of an independent foam. Further, an adhesive sheet from which resin does not remain even when peeled may be used as the detachable suction sheet 31. With such a suction sheet 31, the close-up imaging device 10 can be detachably provided on the surface 51 of the smartphone. As a result, movement on the surface 51 of the smartphone is also easy and, as illustrated in Fig. 7A, the base plate 1 can be slid to arrange the opening 9 of the base plate 1 in front of the light source 52 or, as illustrated in Fig. 7B, the base plate 1 can be slid to arrange the color filter 4 that also serves as the base plate 1 in front of the light source 52.

### (Base plate / color filter)

As illustrated in Fig. 1 and the like, the base plate 1 is a component of the close-up imaging device 10 of the first embodiment, and at least the convex lens 2 and the slit light forming part 3 are attached thereto. In the first embodiment illustrated in Fig. 1 and the like, the base plate 1 also serves as the color filter 4, but may not also serve as the color filter 4. When the base plate 1 does not also serve as the color filter 4, the color filter 4 is provided as a separate body from the base plate 1, and examples thereof include slidable attachment (second embodiment described later).

The base plate 1 is a plate having a sheet shape and, as illustrated in Figs. 7A and 7B, is a substantially rectangular member bonded to the surface 51 of the mobile terminal 50 via the suction sheet 31. The base plate 1 is not particularly limited in terms of material, but is preferably made of plastic. A front half of the base plate 1 in a longitudinal direction is a region including the opening 9 that allows the convex lens 2 to be attached to a tip end thereof and light from the light source to pass therethrough. A rear half of the base plate 1 in the longitudinal direction is a region in which the suction sheet 31 is provided. As illustrated in Figs. 7A and 7B, the region of this rear half can be slid while a state in which the convex lens 2 is positioned in front of the imaging camera lens 53 of the mobile terminal 50 is held. This sliding is performed to set the opening 9 in a position that allows the light from the light source 52 to pass therethrough as illustrated in Fig. 7A, and position the base plate 1 also serving as the color filter 4 in front of the light source 52 as illustrated in Fig. 7B.

It should be noted that, with regard to the mobile terminal 50, terminals in which a position and a size of the imaging camera lens 53 and a position and a size of the light source 52 differ for each new model are often released. Therefore, by providing, for example, a sliding position variable device (not illustrated) that enables the slit light forming part 3 to slide in stages, even if the distance between the imaging camera lens 53 and the light source 52 changes, it is possible to adapt to that change. In addition to such adaptation with the sliding position variable device, the design of the close-up imaging device 10 according to the present invention can also be changed as desired in accordance with the positions of the imaging camera lens 53 and the light source 52 of the new model. However, even if there is such a dimensional design change, the components of the close-up imaging device 10 according to the present invention and the roles and the functions played by the components do not change. Accordingly, the covered scope of the present invention can be said to be within the technical scope of the present invention.

The base plate 1 has about the size illustrated in Figs. 7A and 7B and, as long as a plate having a sheet shape having a certain level of strength, is not particularly limited, but preferable examples include various resin plates. When also serving as the color filter 4, the base plate 1 may be a resin plate that also serves as the color filter 4 in its entirety or, as illustrated in Fig. 7B, may be plate to which a resin plate, which functions as the color filter 4 only in a peripheral range including an area that transmits light from the light source 52 when slid, is attached.

The resin plate that serves as the color filter 4 is not particularly limited, but is preferably, for example, a blue filter that converts white light emitted from the light source 52 into blue light. For example, a blue filter that converts white light into blue light having a wavelength of 488 nm is preferred. The blue filter may be a colored acrylic resin, and examples thereof include an acrylic resin blue filter (PGZ 302K 302 manufactured by Kuraray Co., Ltd.), but the filter is not limited thereto. Observation or imaging of an injury of an anterior eye can be performed by covering the light source 52 with such a color filter 4. For example, fluorescein eyedrops are administered to the eye and a blue free filter for vital staining examination is adopted as the color filter 4, making it possible to emit blue light onto the eye to change the color of the injury on the surface of the eye to green, and observe or capture an image of the green light through the imaging camera lens 53. As a result, it is easier to examine keratoconjunctive epithelium disorder and eye injuries, making it possible to observe or capture images of injuries and the like of the anterior eye.

The color filter 4 may be a filter that also serves as the base plate 1 or may be a filter provided as a portion of the base plate 1. The close-up imaging device 10 according to the present invention, by being used to capture an image of or observe the anterior eye through the color filter 4, makes it possible to easily capture an image of or observe an injury of the anterior eye. The color filter 4, as illustrated in Fig. 7A, is removed from in front of the light source 52 during imaging or observation of the anterior eye and, as illustrated in Fig. 7B, is installed in front of the light source 52 during imaging or observation of an injury of the anterior eye. Thus, the color filter 4 can be installed or removed as needed.

When slit light is to be formed using the slit light forming part 3 described later, the color filter 4 is slid to be removed from the optical path of the light source 52 as illustrated in Fig. 7A, and the slit light forming part 3 is slid to be installed in the optical path of the light source 52 as illustrated in Fig. 4. On the other hand, when slit light is not to be formed using the slit light forming part 3, the color filter 4 is slid to be installed in the optical path of the light source 52 as illustrated in Fig. 7B, and the slit light forming part 3 is slid to be removed from the optical path of the light source 52 as illustrated in Fig. 6. Thus, the color filter 4 and the slit light forming part 3 can be installed in or removed from the optical path of the light source 52 by respectively sliding the color filter 4 and the slit light forming part 3.

### (Convex lens)

When the close-up imaging device 10 is mounted to the mobile terminal 50, the convex lens 2 is a member positioned in front of the imaging camera lens 53. The convex lens 2 is selected as desired in consideration of the focal length at which the internal state and the surface state of the anterior eye can be observed or captured in an image. The convex lens 2 is adhered or fitted to and thus integrated with the opening 9 provided to the base plate 1. With this convex lens 2, it is possible to adjust the focus on the eye of a mouse or a human, correct blurring of an image, and perform clear observation or imaging.

As illustrated in Figs. 7A and 7B, the convex lens 2 is held in a position in which disengagement from in front of the imaging camera lens 53 does not occur even when the base plate 1 is slid. This way, even when the internal state or the surface state of the anterior eye is observed or captured in an image by slit light, and even when the internal state or the surface state of the anterior eye is observed or captured in an image by light transmitted through the color filter 4, the observation or imaging can be performed.

### (Slit light forming part)

When the close-up imaging device 10 is mounted to the mobile terminal 50, the slit light forming part 3 is a member positioned in front of the light source 52. The slit light forming part 3 is a member that forms the light from the light source 52 into slit light by a cylindrical lens 8. The formed slit light reaches the eye, making it possible to observe and capture an image of the internal state of the anterior eye in more detail.

The slit light forming part 3, as illustrated in Fig. 3 and Fig. 4, includes a first reflecting mirror 5 that reflects the light from the light source 52, a second reflecting mirror 6 that reflects light reflected by the first reflecting mirror 5, and a slit part 7 that allows light reflected by the second reflecting mirror 6 to pass therethrough. The light that passes through the slit part 7 becomes slit light by the cylindrical lens 8. Thus, the light reflected by the two reflecting mirrors 5, 6 passes through the slit part 7, making it possible to readily form the slit light. As illustrated in Fig. 1 to Fig. 3, the cylindrical lens 8 is held from both sides in the longitudinal direction.

The first reflecting mirror 5 and the second reflecting mirror 6 are not particularly limited in terms of material or size, but are each desirably constituted by a material and a size that are within a range that limits the size of the slit light forming part 3, and allow light to be guided by the cylindrical lens 8. The cylindrical lens 8 can also be selected and adopted from various cylindrical lenses within a range that limits the size of the slit light forming part 3, in terms of material or size. The slit part 7 is preferably a narrow slit having a minimum width of approximately 0.2 to 1 mm. As the range, the slit part 7 is preferably formed with a width of about 0.15 mm to 1.5 mm. It should be noted that a slit width adjusting member (not illustrated) that can adjust the width of the slit part 7 may be provided. Examples thereof include a slit width adjusting member that narrows or widens the width of the slit part by rotation of a rotary knob.

The cylindrical lens 8, as illustrated in Fig. 5, is slid by a slide member 21 in a direction parallel to the surface of the base plate 1 and in a direction orthogonal to the cylindrical lens 8. By sliding the cylindrical lens 8, it is possible to adjust an incident form in which the slit light is incident on the eye. The slide mechanism is not particularly limited, and may be a structural form that slides by a mechanical eccentric mechanism. It should be noted that the degree of the sliding is several millimeters. It should be noted that reference sign 22 denotes a lever provided to the slide member 21 and, as illustrated in Fig. 5, the cylindrical lens 8 can be slid by rotating this lever 22.

### (Other components)

As another component, as already mentioned, in a case in which the base plate 1 does not also serve as the color filter 4 or in a case in which the color filter 4 is not provided in a portion of the base plate 1, the color filter 4 may be provided as a separate body.

### (Imaging example)

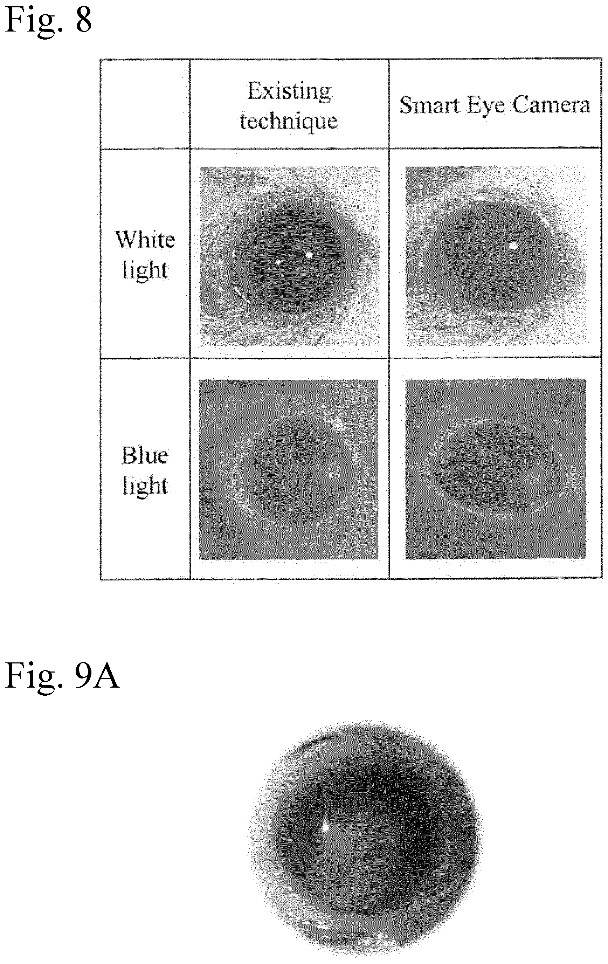

Fig. 8 shows, in the upper row, representative photographs of an eye exposed to white light and, in the lower row, representative photographs of fluorescein staining. In Fig. 8, the images in the left column are examples captured with an existing device, and the images in the right column are examples captured using the close-up imaging device 10. It should be noted that, as the existing device, a device widely used for evaluating the eye of the ED mouse model was used. Specifically, a device constituted by a microscope (product name: SZ61), a camera, and a light source manufactured by Olympus Corporation was used.

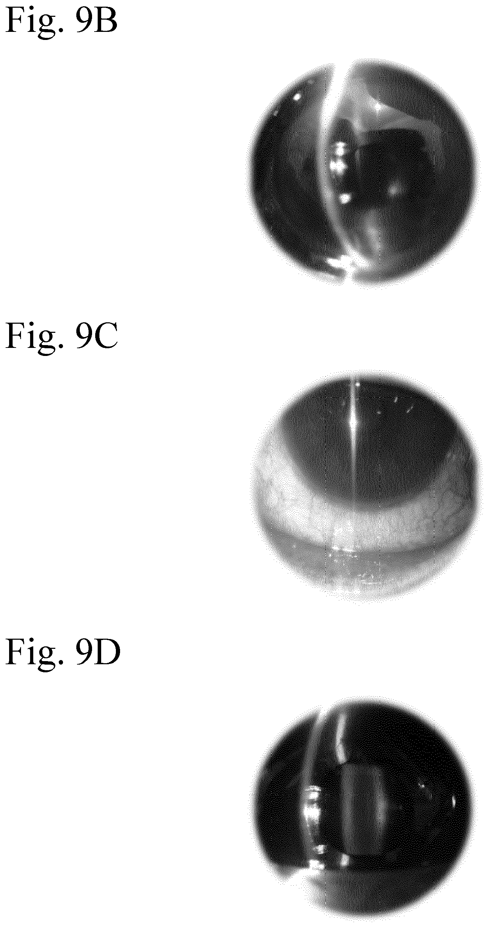

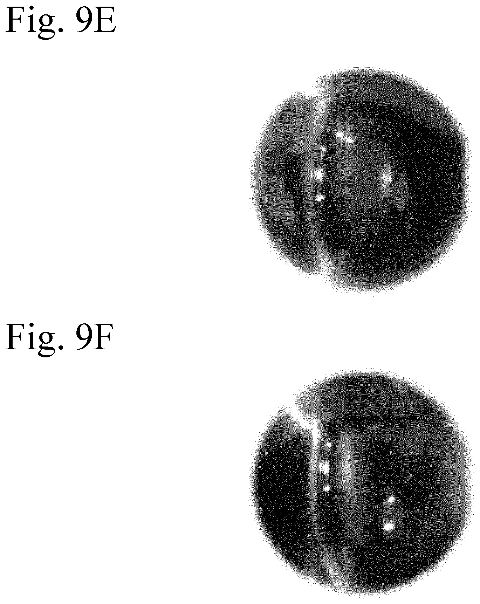

Figs. 9A to 9F are examples of evaluations of nuclear sclerosis (cataracts) by the close-up imaging device 10. With the close-up imaging device 10, evaluation of various anterior eye diseases such as pterygium, corneal opacity, epidemic keratoconjunctivitis, and intraocular lens could be evaluated in all cases. Figs. 9A to 9F are captured images, Fig. 9A showing corneal opacity, Fig. 9B showing post cataract surgery, Fig. 9C showing epidemic conjunctivitis, Fig. 9D showing no cataracts, Fig. 9E showing moderate cataracts, and Fig. 9F showing severe cataracts. The severities of cataracts evaluated with the existing medical device and with the close-up imaging device 10 were the same. Thus, various symptoms of the anterior eye can be observed by the close-up imaging device 10 according to the present invention.

### <Close-up imaging device of second embodiment

The close-up imaging device 10 of the second embodiment is, as illustrated in Figs. 10 and 11, Figs. 12A and 12B, and Figs. 13A and 13B, a close-up imaging device detachably mounted to and used with the mobile terminal 50 equipped with the light source 52 and the imaging camera lens 53. The basic components are the same as those of the first embodiment, except that the first structure 60 including the convex lens 2 and the slit light forming part 3 and the second structure 70 including the convex lens 2 and the color filter 71 are provided as interchangeable members. Then, the engaging block 80 for detachably mounting the interchangeable members (first structure 60 and second structure 70) to the mobile terminal 50 is provided. When mounted to the mobile terminal 50, the close-up imaging device 10 is configured so that the convex lens 2 is positioned in front of the imaging camera lens 53, the slit light forming part 3 is positioned in front of the light source 52, and the color filter 71 is installed or removed from in front of the light source 52. The first structure 60 and the second structure 70 are not particularly limited in terms of material, but are preferably made of plastic.

With this close-up imaging device 10, the interchangeable member (first structure 60 and second structure 70) is mounted by being engaged with the engaging block 80, making it possible to form slit light by the slit light forming part 3 of the first structure 60 and, with the formed slit light reaching the eye, perform observation and imaging of an internal state of an anterior eye in more detail. Further, by observing the anterior eye through the color filter 71 of the second structure 70, it is possible to easily observe injuries of the anterior eye and the like. It should be noted that, in the description of this second embodiment, the components described using the same reference signs as those of the first embodiment are the same as the components of the first embodiment, and thus description thereof may be omitted.

### (First structure)

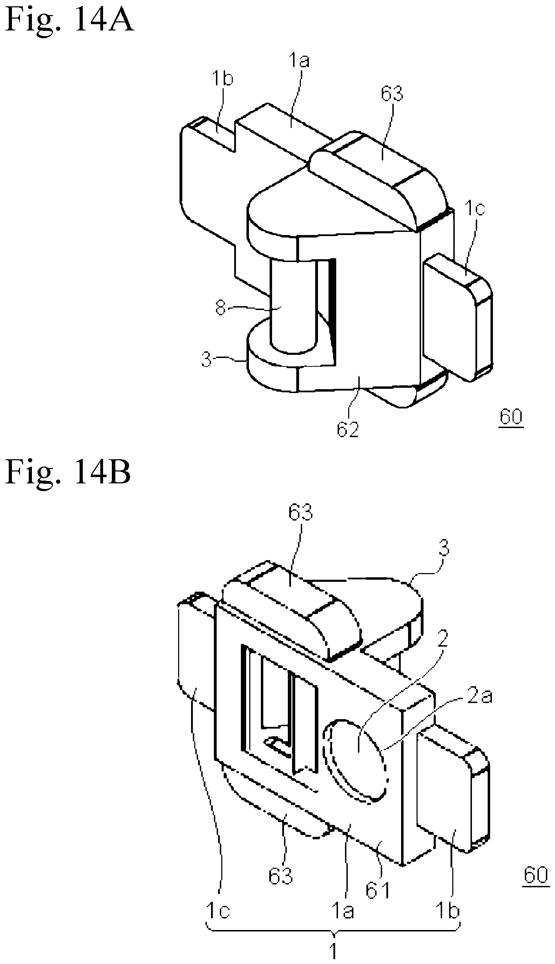

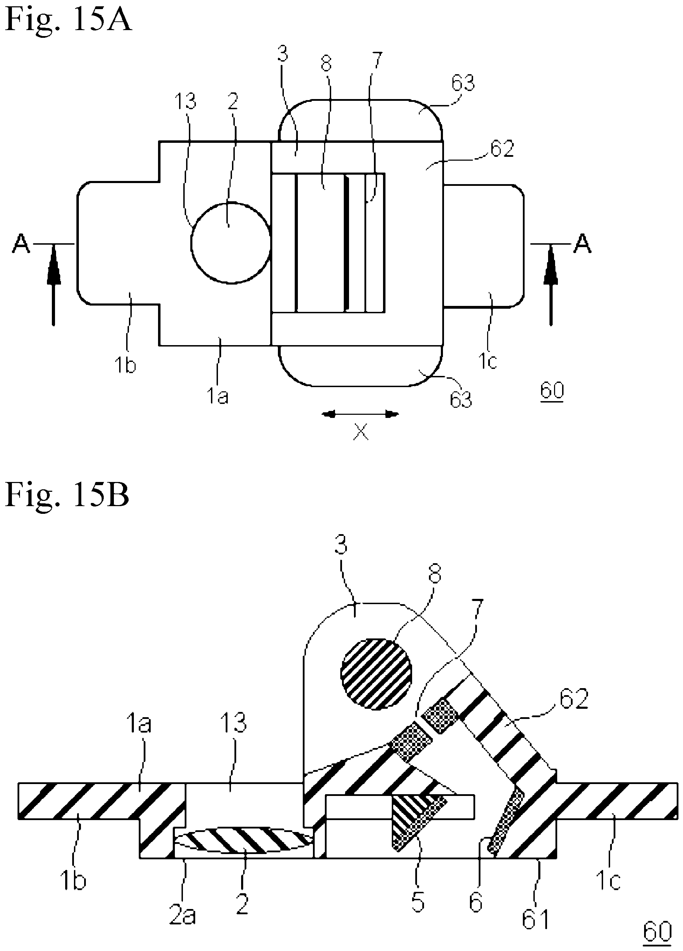

As illustrated in Fig. 10, Fig. 11, Figs. 14A and 14B, and Figs. 15A and 15B, the first structure 60 is an interchangeable member including the convex lens 2 and the slit light forming part 3. The first structure 60 includes the base plate 1, and the base plate 1 is provided with the convex lens 2 and the slit light forming part 3. The base plate 1 includes a base main body part 1a and a tip end engaging protruding part 1b and/or a rear end engaging protruding part 1c. The tip end engaging protruding part 1b and the rear end engaging protruding part 1c may both be provided as illustrated in Figs. 14A and 14B and Figs. 15A and 15B, or only one may be provided. Whether the engaging protruding part is provided at both end portions or one end portion of the base main body part 1a is as desired, but is preferably provided to both end portions to make it possible to adapt to differences in structure of the various mobile terminals 50 (that is, a case in which the arrangement relationship between the light source 52 and the imaging camera lens 53 is as in Fig. 10 or a case of the reverse). It should be noted that the "tip end" and "rear end" have no particular meaning, such that the side on which the convex lens 2 is provided is referred to as the "tip end."

Either the tip end engaging protruding part 1b or the rear end engaging protruding part 1c engages with an engaging recessed part 81 of the engaging block 80. By the engagement, the first structure 60 and the second structure 70 can be interchangeably mounted to the mobile terminal 50. The tip end engaging protruding part 1b and the rear end engaging protruding part 1c are not particularly limited in terms of shape and size, and are designed in accordance with a shape and a size of the engaging recessed part 81 of the engaging block 80, which is the engaging counterpart.

The base main body part 1a is provided with at least the convex lens 2 and the slit light forming part 3. The convex lens 2 is attached to an attaching hole 2a provided to a mounting surface 61 on the tip end engaging protruding part 1b side of the base main body part 1a. The attaching hole 2a is integrated with an opening 13. The opening 13 allows light for capturing an image by the imaging camera lens 53 to pass therethrough. The mounting surface 61 is a surface by which the base plate 1 comes into contact with the mobile terminal 50. It should be noted that the convex lens 2 is the same as that described in the first embodiment.

The slit light forming part 3, similar to that in the first embodiment, is a member that forms the light from the light source 52 into slit light by the cylindrical lens 8. Then, similar to the first embodiment, the slit light forming part 3 includes the first reflecting mirror 5 that reflects the light from the light source 52, the second reflecting mirror 6 that reflects light reflected by the first reflecting mirror 5, and the slit part 7 that allows light reflected by the second reflecting mirror 6 to pass therethrough, and is a member that forms the light that passes through the slit part 7 into slit light by the cylindrical lens 8. The cylindrical lens 8 is held by a cylindrical lens holding member 62 and mounted to the base plate 1.

The slit light forming part 3, similar to that in the first embodiment, preferably includes a slide function capable of sliding in accordance with the distance between the light source 52 of the mobile terminal 50 and the imaging camera lens 53. This slide mechanism is not essential, but is preferably provided. The sliding operation can be performed by pinching slide members 63 provided on both sides of the base plate 1 by hand. "Both sides" refers to sides in the axial direction of the cylindrical lens 8. The slide mechanism is not particularly limited, but can be installed by various methods such as engaging a recessed rail and a protruding rail extending in a slide direction X on both sides. It should be noted that the slit light forming part 3 as well as the cylindrical lens 8, the first reflecting mirror 5, the second reflecting mirror 6, and the slit part 7 constituting the slit light forming part 3 are also the same as those described in the first embodiment.

### (Second structure)

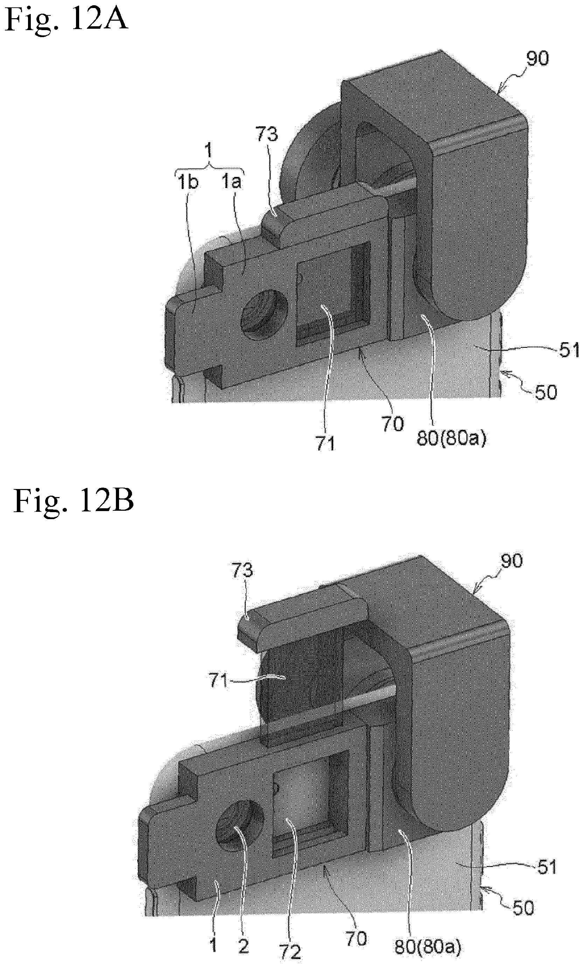

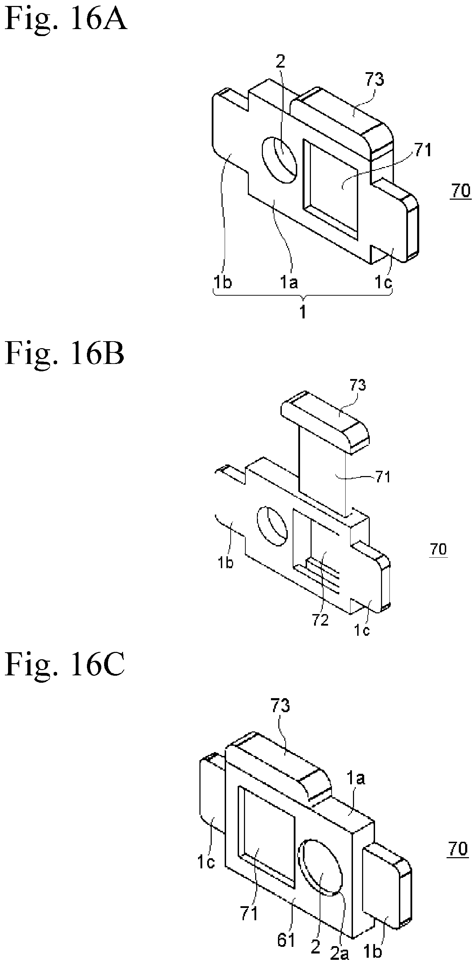

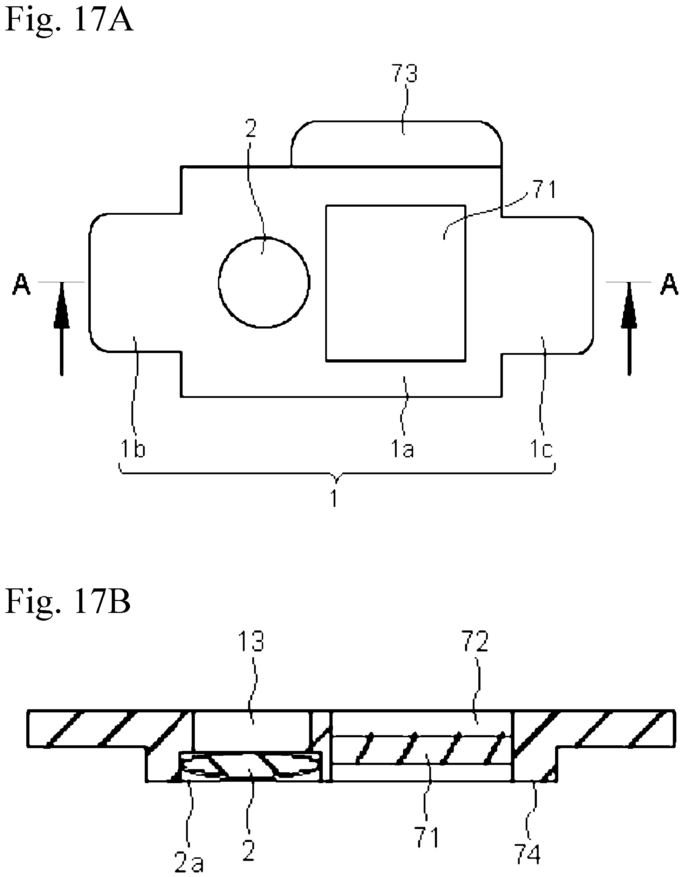

As illustrated in Figs. 12A and 12B, Figs. 13A and 13B, Figs. 16A to 16C, and Figs. 17A and 17B, the second structure 70 is an interchangeable member including the convex lens 2 and the color filter 71. The second structure 70 also includes, similar to the first structure 60, the base plate 1, and the base plate 1 is provided with the convex lens 2 and the color filter 71. The base plate 1 as well as the base main body part 1a, the tip end engaging protruding part 1b, and the rear end engaging protruding part 1c constituting the base plate 1 are the same as those described in the explanation section of the first structure 60, and thus description thereof will be omitted.

The base main body part 1a includes an opening window 72 on the light source 52, and the color filter 71 is installably and removably provided to the opening window 72. The illustrated opening window 72 is rectangular, but may be circular or elliptical, and a shape thereof is not particularly limited. The color filter 71 illustrated in Figs. 12A and 12B and the like is provided as a slide plate inserted into a long hole made in a side surface of the base main body part 1a, but may be provided as an opening/closing door that can be opened and closed by a hinge or the like. Such a color filter 71 is removed from the opening window 72 during imaging or observation of an anterior eye, and installed in the opening window 72 during imaging or observation of an injury of an anterior eye. It should be noted that reference sign 73 denotes a gripping part for gripping the color filter 71 by hand for sliding, and reference sign 74 denotes a mounting surface when the second structure 70 is mounted to the mobile terminal 50.

### (Engaging block)

The engaging block 80 includes the engaging recessed part 81. The engaging recessed part 81 engages with the engaging protruding part (tip end engaging protruding part 1b or rear end engaging protruding part 1c) of the first structure 60, or the engaging protruding part (tip end engaging protruding part 1b or rear end engaging protruding part 1c) of the second structure 70. By the engagement, the first structure 60 and the second structure 70 can be interchangeably mounted to the mobile terminal 50. The engaging recessed part 81 is not particularly limited in terms of shape and size, and is designed in accordance with a shape and a size of the engaging protruding part (tip end engaging protruding part 1b or rear end engaging protruding part 1c), which is the engaging counterpart.

Examples of the engaging block 80 include, as illustrated in Fig. 10, Figs. 12A and 12B, and Figs. 20A to 20D, a clamp engaging block 80a mounted to the mobile terminal 50 by a clamp 90 having a C shape or an adhering engaging block 80b mounted to the mobile terminal 50 by an adhesive member 83. A shape of the engaging block 80 is a rectangular block structure, and a thickness of the engaging block 80 is not particularly limited, but is preferably about the same thickness as that of the base plate 1 of the first structure 60 and the second structure 70.

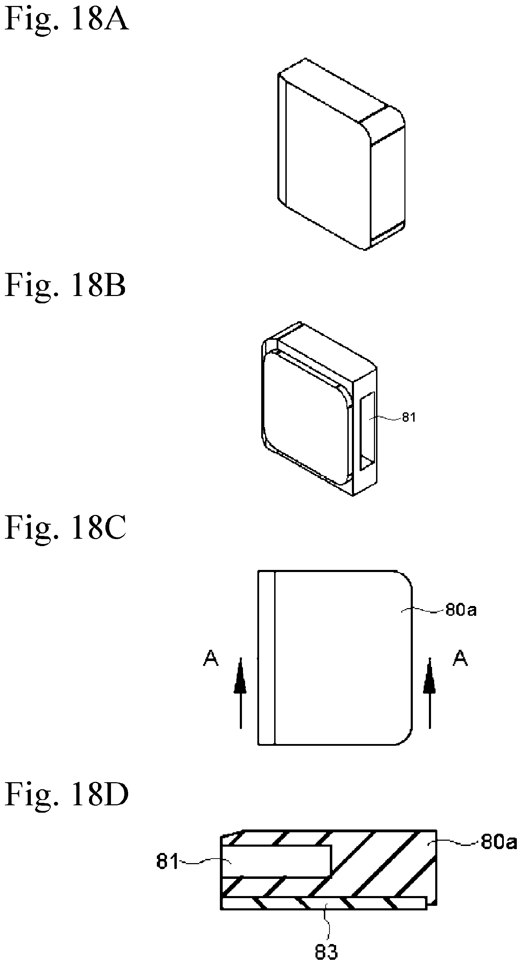

The clamp engaging block 80a has a block-like form as illustrated in Figs. 18A to 18D and, on one side surface thereof, is provided with the engaging recessed part 81 having an elongated hole shape that engages with the tip end engaging protruding part 1b or the rear end engaging protruding part 1c described above. Preferably, an elastic member (rubber, for example) 82 having a predetermined thickness is provided on the side where this clamp engaging block 80a is mounted to the mobile terminal 50. The elastic member 82 acts to prevent slippage and scratches.

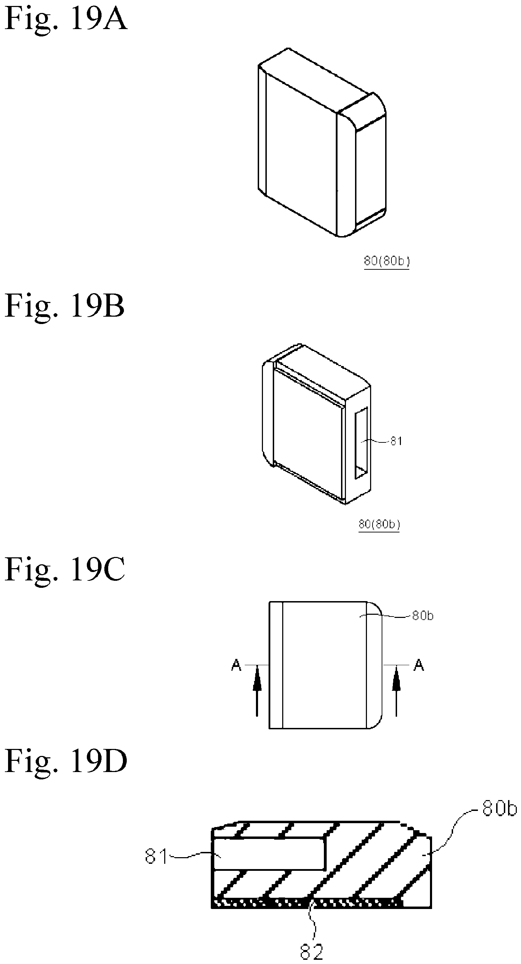

The adhering engaging block 80b has a block-like form as illustrated in Figs. 19A to 19C and, similar to the clamp engaging block 80a, on one side surface thereof, is provided with the engaging recessed part 81 having an elongated hole shape that engages with the tip end engaging protruding part 1b or the rear end engaging protruding part 1c described above. Preferably, the adhesive member (adhesive gel, for example) 83 having a predetermined thickness is provided on the side where this adhering engaging block 80b is mounted to the mobile terminal 50. This adhesive member 83 can be adhered and fixed to the mobile terminal 50.

### (Clamp)

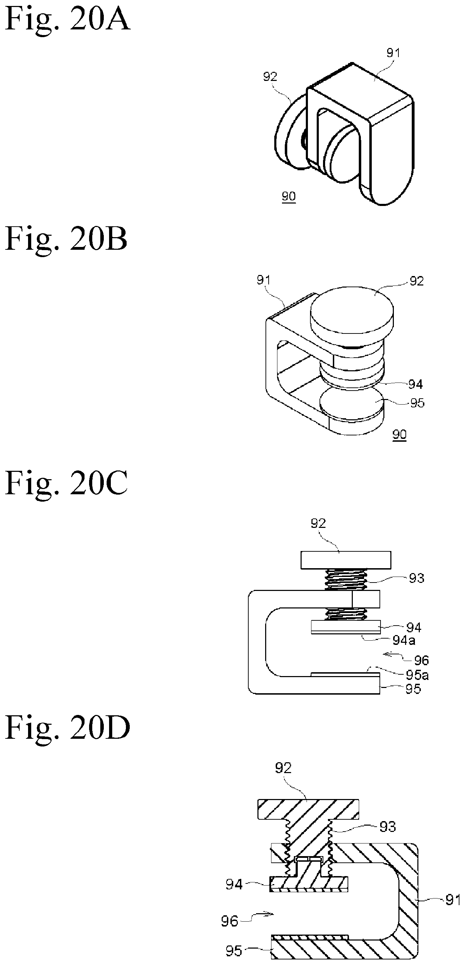

The clamp 90 is used only in a case in which the clamp engaging block 80a is used. The form of the clamp 90 is not particularly limited, but as illustrated in Figs. 20A to 20D, examples include a clamp constituted by a C-shaped main body part 91, a knob part 92, a screw part 93, contacting parts 94, 95, and elastic members (rubber, for example) 94a, 95a provided on surfaces of the contacting parts. The mobile terminal 50 and the engaging block 80 enter and are fixed in a clamp space 96 interposed between the contacting parts 94, 95.

### (Imaging example)

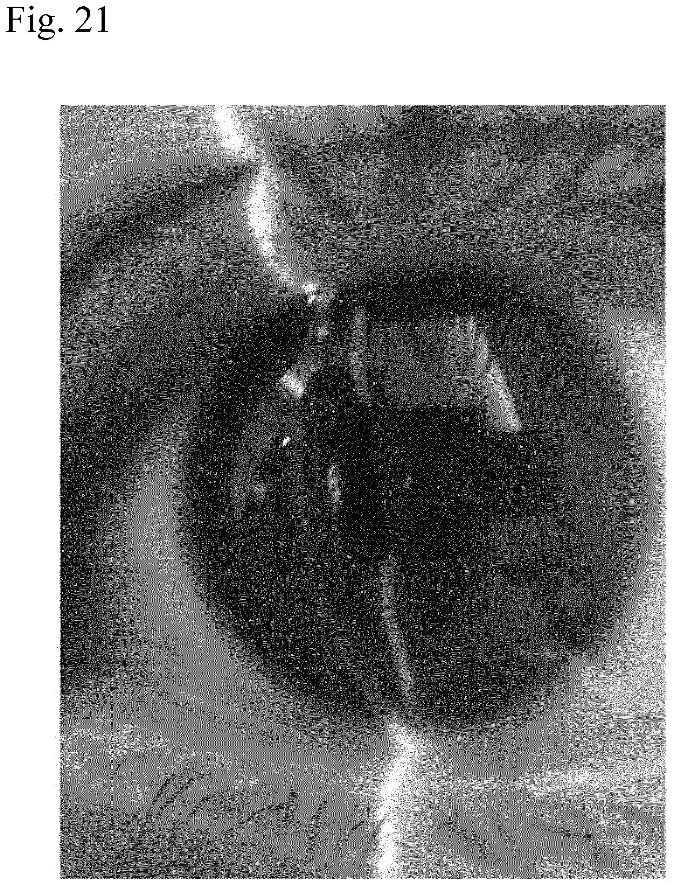

Fig. 21 is a representative photograph of an eye exposed to white slit light by using the close-up imaging device 10 of the second embodiment.

### (Effects and the like of close-up imaging device)

The close-up imaging device 10 according to the present invention described above can be easily mounted to the mobile terminal 50, such as a smartphone which is used worldwide, as an external attachment. This makes it possible to capture all still images and moving images obtained from an ophthalmic examination of a patient, that is, still images and moving images of an internal state and a surface state of an anterior eye. Furthermore, the close-up imaging device 10 is significantly inexpensive compared to a slit lamp microscope or a handheld slit lamp microscope, making it easy to prepare a plurality of devices and, by using a device prepared for animals separately from human clinical use, it is possible to capture still images and moving images of the eyes of experimental animals (animal models), companion animals such as pets, and reared animals in zoos and acquire ophthalmic findings of the above-described animals as well.

Further, clinical use is possible. Specifically, an ophthalmic examination is performed with the patient fixed onto a provided stand. Therefore, examinations for children and bedridden elderly require expert skills. However, the close-up imaging device 10 according to the present invention adds the element of "recording" to the portable medical instrument, and can be used to share recorded still images and moving images among medical personnel. The shared data can be expected to be utilized in remote medical care in rural areas and support for developing countries, and can be further expected to improve a diagnostic accuracy of ophthalmologists by being analyzed by artificial intelligence (AI) as big data. Then, finally, the close-up imaging device 10 is used as a self-diagnostic tool by all smartphone users, making it possible to further develop the ophthalmic examination itself.

Further, the close-up imaging device 10 according to the present invention can be utilized for research. Specifically, the findings of the eyes of research animals to date could not be substantially recorded due to various technical problems (skillful technique is required, the target animal must be killed during imaging, imaging instrument is expensive, and the like). However, by using the close-up imaging device 10 according to the present invention, it is possible to easily obtain eye findings of research animals, and thus expect birth of a new phenotype in the research field of the eye.

Further, the close-up imaging device 10 according to the present invention is conceivably used in an ophthalmic examination, but is not limited to being used in an ophthalmic examination. For example, the close-up imaging device 10 may be used not only in an ophthalmic outpatient clinic, but also in a place other than an ophthalmic clinic, such as a medical examination place, a long-term care health facility for the elderly, an ambulance, and a science or health or medical school class. In addition, the close-up imaging device 10 can be used in animal-related facilities such as an animal hospital or a zoo health center or a research institute as well.

### Descriptions of Reference Numerals

- 10: Close-up imaging device
- 1: Base plate
- 1a: Base main body part
- 1b: Tip end engaging protruding part
- 1c: Rear end engaging protruding part
- 2: Convex lens
- 2a: Attaching hole
- 3: Slit light forming part
- 4: Color filter
- 4a: Color filter
- 5: First reflecting mirror
- 6: Second reflecting mirror
- 7: Slit
- 8: Cylindrical lens
- 9: Opening
- 11: Base plate
- 12: Structure
- 13: Opening window
- 21: Slide member
- 22: Lever
- 31: Suction sheet
- 50: Mobile terminal (Smartphone)
- 51: Mounting surface
- 52: Light source
- 53: Imaging camera lens
- 60: First structure
- 61: Mounting surface
- 62: Cylindrical lens holding member
- 63: Slide member
- X: Slide direction
- 70: Second structure
- 71: Color filter
- 72: Opening window
- 73: Gripping part
- 74: Mounting surface
- 80: Engaging block
- 80a: Clamp engaging block
- 80b: Adhering engaging block
- 81: Engaging recessed part
- 82: Elastic member (Rubber)
- 83: Adhesive member (Adhesive gel)
- 90: Clamp
- 91: C-shaped main body part
- 92: Knob part
- 93: Screw part
- 94, 95: Contacting part
- 94a, 95a: Elastic member (Rubber)
- 96: Clamp space

## Claims

1. A close-up imaging device detachably mounted to and used with a mobile terminal equipped with a light source and an imaging camera lens, the close-up imaging device being an integral structure comprising at least:
a convex lens;
a slit light forming part; and
a color filter,
when the close-up imaging device is mounted to the mobile terminal, the convex lens being a member positioned in front of the imaging camera lens, the slit light forming part being a member positioned in front of the light source, and the color filter being a member installed or removed from in front of the light source.

2. The close-up imaging device according to claim 1, wherein
the color filter is a member removed from in front of the light source during imaging or observation of an anterior eye, and installed in front of the light source during observation or imaging of an injury of an anterior eye.

3. The close-up imaging device according to claim 1 or 2, the close-up imaging device being an integral structure further comprising:
a base plate positioned on the mobile terminal side, and
the color filter also serving as the base plate or provided as a portion of the base plate, and
when slit light is to be formed by the slit light forming part, the color filter being slid to be removed from an optical path of the light source, and the slit light forming part being slid to be installed in the optical path, and
when slit light is not to be formed by the slit light forming part, the color filter being slid to be installed in the optical path, and the slit light forming part being slid to be removed from the optical path.

4. The close-up imaging device according to any one of claims 1 to 3, wherein
the slit light forming part is a member that forms light from the light source into slit light by a cylindrical lens.

5. The close-up imaging device according to any one of claims 1 to 3, wherein
the slit light forming part includes a first reflecting mirror that reflects the light from the light source, a second reflecting mirror that reflects light reflected by the first reflecting mirror, and a slit part that allows light reflected by the second reflecting mirror to pass therethrough, and is a member that forms light that passes through the slit part into slit light by the cylindrical lens.

6. The close-up imaging device according to claim 4 or 5, further comprising a slide member for sliding the cylindrical lens in a direction parallel to the base plate and in a direction orthogonal to the cylindrical lens.

7. A close-up imaging device detachably mounted to and used with a mobile terminal equipped with a light source and an imaging camera lens, the close-up imaging device comprising:
a first structure including a convex lens and a slit light forming part, and a second structure including a convex lens and a color filter, as interchangeable members; and
an engaging block detachably mounted to the mobile terminal and for engaging the interchangeable members,
when the close-up imaging device is mounted to the mobile terminal, the convex lens being positioned in front of the imaging camera lens, the slit light forming part being positioned in front of the light source, and the color filter being installed or removed from in front of the light source.

8. The close-up imaging device according to claim 7, wherein
the slit light forming part is a member that forms light from the light source into slit light by a cylindrical lens.

9. The close-up imaging device according to claim 7 or 8, wherein
the slit light forming part includes a first reflecting mirror that reflects the light from the light source, a second reflecting mirror that reflects light reflected by the first reflecting mirror, and a slit part that allows light reflected by the second reflecting mirror to pass therethrough, and is a member that forms light that passes through the slit part into slit light by the cylindrical lens.

10. The close-up imaging device according to any one of claims 7 to 9, wherein
the slit light forming part further includes a slide function capable of sliding in accordance with a distance between the light source of the mobile terminal and the imaging camera lens.

11. The close-up imaging device according to any one of claims 7 to 10, wherein
the second structure further includes an opening window on the light source, and the color filter is a member removed from the opening window during imaging or observation of an anterior eye, and installed in the opening window during observation or imaging of an injury of an anterior eye.

12. The close-up imaging device according to any one of claims 7 to 11, wherein
the engaging block includes an engaging recessed part, and the engaging recessed part engages with an engaging protruding part provided to both the first structure and the second structure.

13. The close-up imaging device according to any one of claims 7 to 12, wherein
the engaging block is a clamp engaging block mounted to the mobile terminal by a C-clamp or an adhering engaging block mounted to the mobile terminal by an adhesive member.
